(19) Europäisches Patentamt
European Patent Office
Office européen des brevets

(11) EP 0 743 971 B1

(12) **EUROPÄISCHE PATENTSCHRIFT**

(45) Veröffentlichungstag und Bekanntmachung des Hinweises auf die Patenterteilung:
**03.09.1997 Patentblatt 1997/36**

(21) Anmeldenummer: **95908219.9**

(22) Anmeldetag: **30.01.1995**

(51) Int Cl.$^6$: **C09K 19/34**, C07D 221/12

(86) Internationale Anmeldenummer:
**PCT/EP95/00312**

(87) Internationale Veröffentlichungsnummer:
**WO 95/21227 (10.08.1995 Gazette 1995/34)**

(54) **PHENANTHRIDIN-DERIVATE UND IHRE VERWENDUNG IN FLÜSSIGKRISTALLINEN MISCHUNGEN**

PHENANTHRIDINE DERIVATIVES AND THEIR USE IN LIQUID CRYSTAL MIXTURES

DERIVES DE PHENANTHRIDINE ET LEUR UTILISATION DANS DES MELANGES DE CRISTAUX LIQUIDES

(84) Benannte Vertragsstaaten:
**DE FR GB**

(30) Priorität: **01.02.1994 DE 4402986**

(43) Veröffentlichungstag der Anmeldung:
**27.11.1996 Patentblatt 1996/48**

(73) Patentinhaber: **HOECHST AKTIENGESELLSCHAFT**
**65929 Frankfurt am Main (DE)**

(72) Erfinder:
- **WINGEN, Rainer**
  **D-65795 Hattersheim (DE)**
- **HORNUNG, Barbara**
  **D-63594 Hasselroth (DE)**

(56) Entgegenhaltungen:
- **PATENT ABSTRACTS OF JAPAN vol. 5 no. 195 (C-083) ,11.Dezember 1981 & JP,A,56 118070 (KANEOKA YUICHI) 16.September 1981,**
- **PATENT ABSTRACTS OF JAPAN Bd. 17, Nr. 232 (C-1056) 12 Mai 1993 & JP,A,04 364 128 (NIPPON KAYAKU) 16 Dezember 1992**
- **PATENT ABSTRACTS OF JAPAN Bd. 17, Nr. 533 (C-1114) 27 September 1993 & JP,A,05 148 239 (SUZUKI MASONABU) 15 Juni 1993**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 743 971 B1

## Beschreibung

Neben nematischen und cholesterischen Flüssigkristallen werden in jüngerer Zeit auch optisch aktive geneigt smektische (ferroelektrische) Flüssigkristalle in kommerziellen Displayvorrichtungen verwendet.

Clark und Lagerwall konnten zeigen, daß der Einsatz ferroelektrischer Flüssigkristalle (FLC) in sehr dünnen Zellen zu optoelektrischen Schalt- oder Anzeigeelementen führt, die im Vergleich zu den herkömmlichen TN (twisted nematic")-Zellen um bis zu einem Faktor 1000 schnellere Schaltzeiten haben (siehe z.B. EP-A 0 032 362). Aufgrund dieser und anderer günstiger Eigenschaften, z.B. der bistabilen Schaltmöglichkeit und des nahezu blickwinkelunabhängigen Kontrasts, sind FLCs grundsätzlich für Anwendungsgebiete wie Computerdisplays gut geeignet.

Für die Verwendung von FLCs in elektrooptischen oder vollständig optischen Bauelementen benötigt man entweder Verbindungen, die geneigte bzw. orthogonale smektische Phasen ausbilden und selbst optisch aktiv sind, oder man kann durch Dotierung von Verbindungen, die zwar solche smektischen Phasen ausbilden, selbst aber nicht optisch aktiv sind, mit optisch aktiven Verbindungen ferroelektrische smektische Phasen induzieren. Die gewünschte Phase soll dabei über einen möglichst großen Temperaturbereich stabil sein.

Zur Erzielung eines guten Kontrastverhältnisses in elektrooptischen Bauelementen ist eine einheitliche planare Orientierung der Flüssigkristalle nötig. Eine gute Orientierung in der $S_A$ und $S^*_C$-Phase läßt sich z.B. erreichen, wenn die Phasenfolge der Flüssigkristallmischung mit abnehmender Temperatur lautet:

$$\text{Isotrop} \rightarrow N^* \rightarrow S_A \rightarrow S^*_C$$

Vorraussetzung ist, daß der Pitch (Ganghöhe der Helix) in der N*-Phase sehr groß (größer 10 µm) oder, noch besser, völlig kompensiert ist (siehe z.B. T. Matsumoto et al., p. 468-470, Proc. of the 6th Int. Display Research Conf., Japan Display, Sept. 30 - Okto. 2, 1986, Tokyo, Japan; M. Murakami et al., ibid. S. 344 - S. 347). Dies erreicht man, z.B. indem man zu der chiralen Flüssigkristallmischung, die in der N*-Phase z.B. eine linksdrehende Helix aufweist, einen oder mehrere optisch aktive Dotierstoffe, die eine rechtsdrehende Helix induzieren, in solchen Mengen hinzugibt, daß die Helix kompensiert wird.

Für die Verwendung des SSFLCD-Effektes (Surface Stabilized Ferroelectric Liquid Crystal Display) von Clark und Lagerwall zur einheitlichen, planaren Orientierung ist ferner Vorraussetzung, daß der Pitch in der smektischen C* Phase wesentlich größer ist als die Dicke des Anzeigeelementes (Mol. Cryst. Liq. Cryst. 94 (1983) 213-134 und 114 (1984) 151-187). Dies erreicht man, wie im Fall des cholesterischen Pitches, durch Verwendung von Dotierstoffen mit entgegengesetztem Drehsinn der Helix.

Die optische Schaltzeit $\tau$ [µs] ferroelektrischer Flüssigkristallsysteme, die möglichst kurz sein soll, hängt von der Rotationsviskosität des Systems $\gamma$ [mPas], der spontanen Polarisation $P_s$[nC/cm$^2$] und der elektrischen Feldstärke E [V/m] ab nach der Beziehung

$$\tau \sim \frac{\gamma}{P_s \cdot E}$$

Da die Feldstärke E durch den Elektrodenabstand im elektrooptischen Bauteil und durch die angelegte Spannung festgelegt ist, muß das ferroelektrische Anzeigemedium niedrigviskos sein und eine hohe spontane Polarisation aufweisen, damit eine kurze Schaltzeit erreicht wird.

Schließlich wird neben thermischer, chemischer und photochemischer Stabilität eine kleine optische Anisotropie $\Delta n$, vorzugsweise $\approx 0,13$, und eine geringe positive oder vorzugsweise negative dielektrische Anisotropie $\Delta\varepsilon$ verlangt (siehe z.B. S.T. Lagerwall et al., "Ferroelectric Liquid Crystals for Displays" SID Symposium, Oct. Meeting 1985, San Diego, Ca, USA).

Die Gesamtheit dieser Forderungen ist nur mit Mischungen aus mehreren Komponenten zu erfüllen. Als Basis (oder Matrix) dienen dabei bevorzugt Verbindungen, die möglichst selbst bereits die gewünschte Phasenfolge $I \rightarrow N \rightarrow S_A \rightarrow S_C$ aufweisen. Weitere Komponenten der Mischung werden oftmals zur Schmelzpunktserniedrigung und zur Verbreiterung der $S_C$- und meist auch N-Phase, zum Induzieren der optischen Aktivität, zur Pitch-Kompensation und zur Anpassung der optischen und dielektrischen Anisotropie zugesetzt, wobei aber beispielsweise die Rotationsviskosität möglichst nicht vergrößert werden soll.

Ferroelektrische Flüssigkristallanzeigen lassen sich auch durch Nutzung des DHF (Distorted Helix Formation)-Effektes oder des PSFLCD-Effektes (Pitch Stabilized Ferroelectric Liquid Crystal Display, auch SBF = Short pitch Bistable Ferroelektric Effekt genannt) betreiben. Der DHF-Effekt wurde von B.I. Ostrovski in Advances in Liquid Crystal Research and Applications, Oxford/Budapest 1980, 469 ff. beschrieben, der PSFLCD-Effekt ist in DE-A 39 20 625 bzw. EP-A O 405 346 beschrieben. Zur Nutzung dieser Effekte wird im Gegensatz zum SSFLCD-Effekt ein flüssigkristallines Material mit einem kurzen $S_C$-Pitch benötigt.

Derivate des Phenanthrens (wozu hier auch 9,10-Dihydrophenanthrene gezählt werden) wurden bereits als Flüssigkristalle bzw. als Komponenten flüssigkristalliner Mischungen beschrieben:
Azomethine mit einer Phenanthren- bzw. 9,10-Dihydrophenanthren-Einheit (J. Chem. Soc. [London] (1958) 552; J. Chem. Soc., Perkin II (1982) 465); Keto-Derivate des 9,10-Dihydrophenanthrens bzw. Phenanthrens (Chem. Ind. [London] (1974) 615; Prod. Int. Liq. Cryst. Conf. (1973) 397; Tetrahedron 37, 2815 (1981)); Carboxyl-Derivate des 9,10-Dihydrophenanthrens (DD-WP 153 826); 2,7-Bis(alkyloxy)phenanthrene (Nippon Kagaku Kaishi (1980) 250) sowie 9,10-Dihydrophenanthrene mit mesogenen Resten in 2,7-Position (JP 05,262,744).

Da die Entwicklung, insbesondere von ferroelektrischen Flüssigkristallmischungen, noch in keiner Weise als abgeschlossen betrachtet werden kann, sind die Hersteller von Displays an den unterschiedlichsten Komponenten für Mischungen interessiert. Dieses u.a. auch deshalb, weil erst das Zusammenwirken der flüssigkristallinen Mischungen mit den einzelnen Bauteilen der Anzeigevorrichtung bzw. der Zellen (z.B. der Orientierungsschicht) Rückschlüsse auf die Qualität auch der flüssigkristallinen Mischungen zuläßt.

Aufgabe der vorliegenden Erfindung war es daher, neue Verbindungen bereitzustellen, die in flüssigkristallinen Mischungen geeignet sind, das Eigenschaftsprofil dieser Mischungen zu verbessern.

Es wurde nun überraschend gefunden, daß bestimmte 3,8-disubstituierte Phenanthridinderivate in besonderer Weise zur Verwendung in ferroelektrischen Flüssigkristallmischungen geeignet sind.

Gegenstand der Erfindung ist eine ferroelektrische Flüssigkristallmischung, enthaltend ein oder mehrere Phenanthridinderivate der Formel (1),

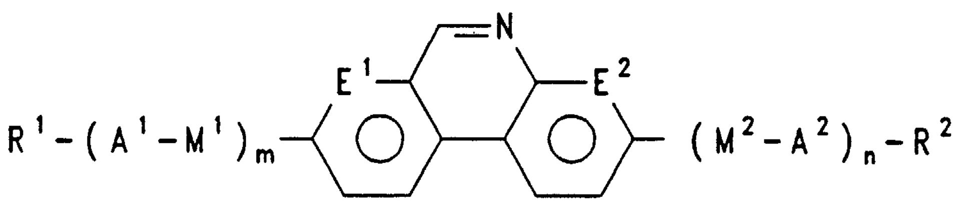

(1)

in der die Symbole und Indizes folgende Bedeutungen haben:

| | |
|---|---|
| $E^1$, $E^2$ | sind gleich oder verschieden -N-, -CF- oder -CH-; |
| $R^1$, $R^2$ | sind gleich oder verschieden ein geradkettiger oder verzweigter Alkylrest mit 1 bis 20 Kohlenstoffatomen (mit oder ohne asymmetrisches Kohlenstoffatom), wobei auch eine oder mehrere $CH_2$-Gruppen durch -O-, -S-, -C≡C-, Cyclopropan-1,2-diyl oder $-Si(CH_3)_2-$ ersetzt sein können, mit der Maßgabe, daß Sauerstoff und Schwefelatome nicht unmittelbar miteinander gebunden sein dürfen, und wobei auch ein oder mehrere Wasserstoffatome des Alkylrests durch Fluor ersetzt sein können, oder eine der nachfolgenden chiralen Gruppen (optisch aktiv oder racemisch): |

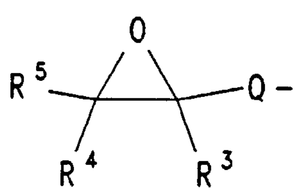
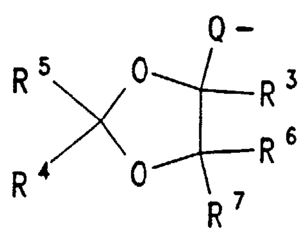
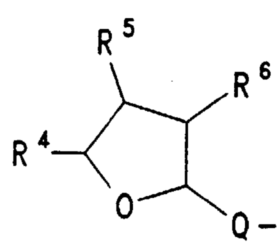

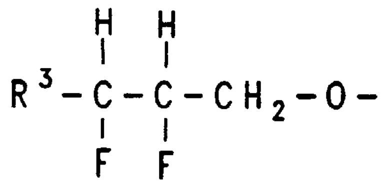

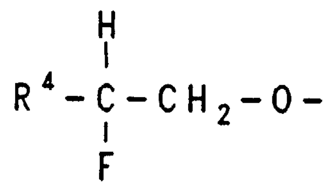

eine der beiden Gruppen $R^1$, $R^2$ kann auch Wasserstoff sein;

$R^3$, $R^4$, $R^5$, $R^6$, $R^7$ sind gleich oder verschieden Wasserstoff oder ein geradkettiger oder verzweigter Alkylrest mit 1 bis 16 Kohlenstoffatomen (mit oder ohne asymmetrisches Kohlenstoffatom), wobei auch eine $CH_2$-Gruppe durch -O- ersetzt sein kann und wobei ein oder mehrere Wasserstoffatome des Alkylrests durch Fluor ersetzt sein können, $R^4$ und $R^5$ können zusammen auch $-(CH_2)_4-$ oder $-(CH_2)_5-$ bedeuten, wenn sie an ein Dioxolansystem gebunden sind;

Q ist $-CH_2$-O-, -CO-O- oder eine Einfachbindung;

$M^1$, $M^2$ sind gleich oder verschieden -CO-O-, -O-CO-, $-CH_2$-O-, -O-$CH_2$-, $-CH_2-CH_2-$, -C≡C- oder eine Einfachbindung;

$A^1$, $A^2$ sind gleich oder verschieden 1,4-Phenylen, wobei ein oder mehrere H-Atome durch F ersetzt sein können, trans-1,4-Cyclohexylen, Pyrimidin-2,5-diyl, Pyridin-2,5-diyl, wobei ein H-Atom durch F ersetzt sein kann, (1,3,4)-Thiadiazol-2,5-diyl, 1,3-Dioxan-2,5-diyl, 1,3-Dithian-2,5-diyl, 1,3-Thiazol-2,4-diyl, 1,3-Thiazol-2,5-diyl, Thiophen-2,4-diyl oder Thiophen-2,5-diyl;

m, n sind Null oder Eins, in der Summe jedoch maximal Eins.

Bereits durch Zusatz geringer Mengen von Phenanthridinderivaten der Formel (I) werden ferroelektrische Flüssigkristallmischungen mit hohen negativen Werten für die dielektrische Anisotropie erhalten.

Bevorzugt enthält die ferroelektrische Flüssigkristallmischung eine oder mehrere Verbindungen der Formel (I) in denen die Gruppe:

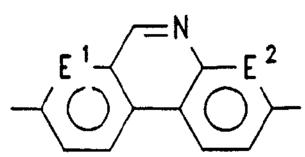

eine der folgenden Bedeutungen hat:

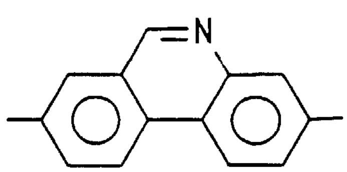

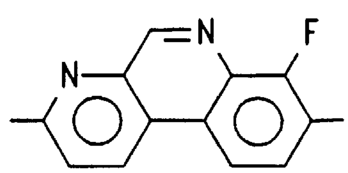

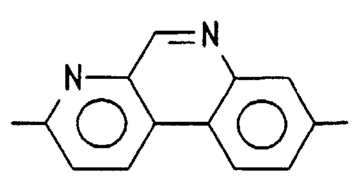

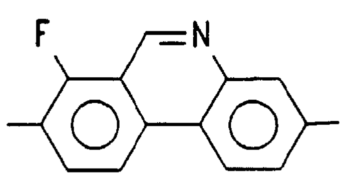

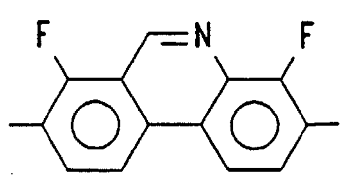

Darunter sind besonders bevorzugt Verbindungen der Formel (I), in denen $R^1$, $R^2$ geradkettige oder verzweigte Alkylreste (mit oder ohne asymmetrisches C-Atom) mit 1 bis 16 C-Atomen sind, wobei auch eine oder mehrere $CH_2$-Gruppen durch -O-, Cyclopropan-1,2-diyl oder $-Si(CH_3)_2-$ ersetzt sein können mit der Maßgabe, daß Sauerstoffatome nicht unmittelbar verbunden sein dürfen, und/oder wobei ein oder mehrere H-Atome der Alkylreste auch durch F substituiert sein können.

Weiterhin besonders bevorzugt für die Verwendung in ferroelektrischen Flüssigkristallmischungen sind die folgenden Gruppen von Verbindungen der Formel (I), in denen $R^1$ und $R^2$ geradkettige oder verzweigte Alkyloxyreste mit 1 bis 10 C-Atomen sind, wobei auch eine durch mindestens zwei weitere $-CH_2$-Gruppen vom Kern getrennte $-CH_2$-Gruppe durch $-Si(CH_3)_2$-ersetzt sein kann:

a) 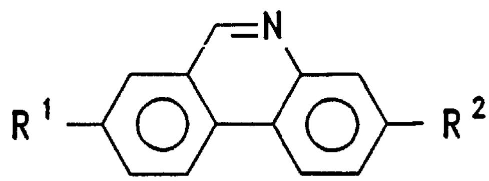

b) 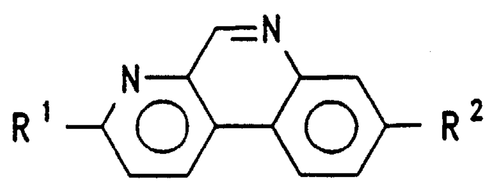

c) 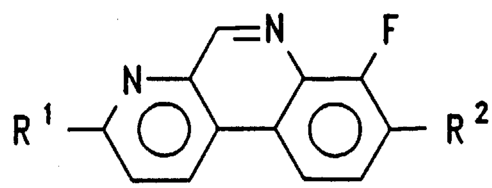

d) 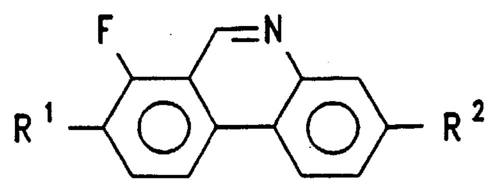

e) 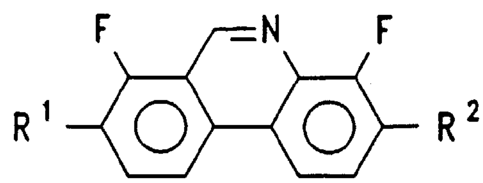

Ebenfalls Gegenstand der Erfindung ist die Verwendung von Phenanthridinderivaten der Formel (I) in ferroelektrischen Flüssigkristallmischungen.

Die Phenanthridinderivate der Formel (II) sind teilweise bekannt und teilweise neu.

Gegenstand der Erfindung sind daher weiterhin Phenanthridinderivate der Formel (Ia),

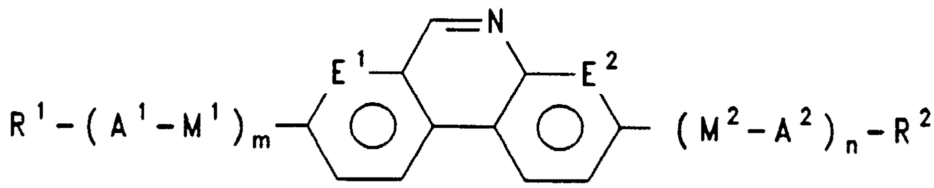

(I a)

in der die Symbole und Indizes folgende Bedeutungen haben:

| | |
|---|---|
| $E^1$, $E^2$ | sind gleich oder verschieden -N-, -CF- oder -CH-; |
| $R^1$, $R^2$ | sind gleich oder verschieden ein geradkettiger oder verzweigter Alkylrest mit 1 bis 20 Kohlenstoffatomen (mit oder ohne asymmetrisches Kohlenstoffatom), wobei auch eine oder mehrere $CH_2$-Gruppen durch -O-, -S-, -C≡C-, Cyclopropan-1,2-diyl oder $-Si(CH_3)_2-$ ersetzt sein können, mit der Maßgabe, daß Sauerstoff und Schwefelatome nicht unmittelbar miteinander gebunden sein dürfen, und wobei auch ein oder mehrere Wasserstoffatome des Alkylrests durch Fluor ersetzt sein können, oder eine der nachfolgenden chiralen Gruppen (optisch aktiv oder racemisch): |

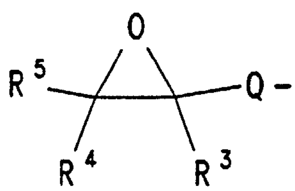
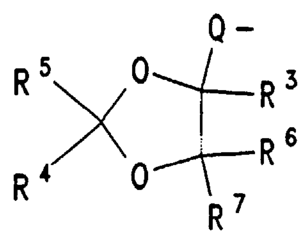
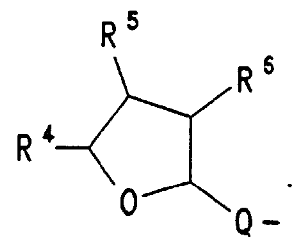

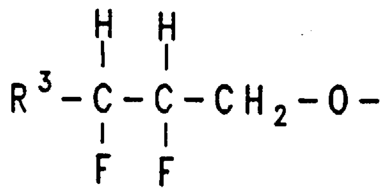
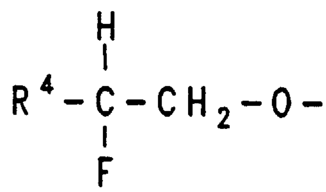

| | |
|---|---|
| $R^3$, $R^4$, $R^5$, $R^6$, $R^7$ | sind gleich oder verschieden Wasserstoff oder ein geradkettiger oder verzweigter Alkylrest mit 1 bis 16 Kohlenstoffatomen (mit oder ohne asymmetrisches Kohlenstoffatom, wobei auch eine $CH_2$-Gruppe durch -O- ersetzt sein kann und wobei ein oder mehrere Wasserstoffatome des Alkylrests durch Fluor ersetzt sein können, $R^4$ und $R^5$ können zusammen auch $-(CH_2)_4-$ oder $-(CH_2)_5-$ bedeuten, wenn sie an ein Dioxolansystem gebunden sind; |
| Q | ist $-CH_2-O-$, -CO-O- oder eine Einfachbindung; |
| $M^1$, $M^2$ | sind gleich oder verschieden -CO-O-, -O-CO-, $-CH_2-O-$, $-O-CH_2-$, $-CH_2-CH_2-$, -C≡C- oder eine Einfachbindung; |
| $A^1$, $A^2$ | sind gleich oder verschieden 1,4-Phenylen, wobei ein oder mehrere H-Atome durch F ersetzt sein können, trans-1,4-Cyclohexylen, Pyrimidin-2,5-diyl, Pyridin-2,5-diyl, wobei ein H-Atom durch F ersetzt sein kann, (1,3,4)-Thiadiazol-2, 5-diyl, 1,3-Dioxan-2,5-diyl, 1,3-Dithian-2,5-diyl, 1,3-Thiazol-2,4-diyl, 1,3-Thiazol-2,5-diyl, Thiophen-2,4-diyl oder Thiophen-2,5-diyl; |
| m, n | sind Null oder Eins, in der Summe jedoch maximal Eins. |

Bevorzugte Phenanthridinderivate der Formel (Ia) entsprechen denen der Formel (I).

Die Herstellung der erfindungsgemäßen Verbindungen erfolgt nach an sich literaturbekannten Methoden, wie sie in Standardwerken zur Organischen Synthese, z. B. Houben-Weyl, Methoden der Organischen Chemie, Georg-Thieme-Verlag, Stuttgart, beschrieben werden.

Die Herstellung erfolgt dabei unter Reaktionsbedingungen, die für die genannten Umsetzungen bekannt und geeignet sind. Dabei kann auch von an sich bekannten, hier nicht näher erwähnten Varianten Gebrauch machen.

Die Ausgangsstoffe können gewünschtenfalls auch in situ gebildet werden, und zwar derart, daß man sie aus dem Reaktionsgemisch nicht isoliert, sondern sofort weiter zu den Verbindungen der Formel (I) umsetzt.

Für die Herstellung von Derivaten des Phenanthridins wird verwiesen auf Advanc. Heterocyclic Chem. 13, 315 (1971); Khim. Geterosikl. Soedin 1969, 1044 (C.A. 1970, 72, 111265 w); Tetrahedron 1981, 37, 825; Zh. Org. Khim. 1978, 14, 891 (C.A. 1978, 89, 24118 h); J. Chem. Soc. Perkin Trans. I, 1972, 113; Org. Reactions 1984, 30, 1; Synthesis 1985, 107; Heterocycles 1980, 14, 1567; Acc. Chem. Res. 1978, 11, 283; Tetrahedron 1984, 40, 1919; Tetrahedron Lett. 29, 5463 (1988); Chem. Scripta 1986, 26, 311.

In den Schemata 1-3 sind derartige literaturbekannte Methoden für drei bevorzugte Verbindungsgruppen der Formeln (I) bzw. (Ia) näher beschrieben.

## Schema 1

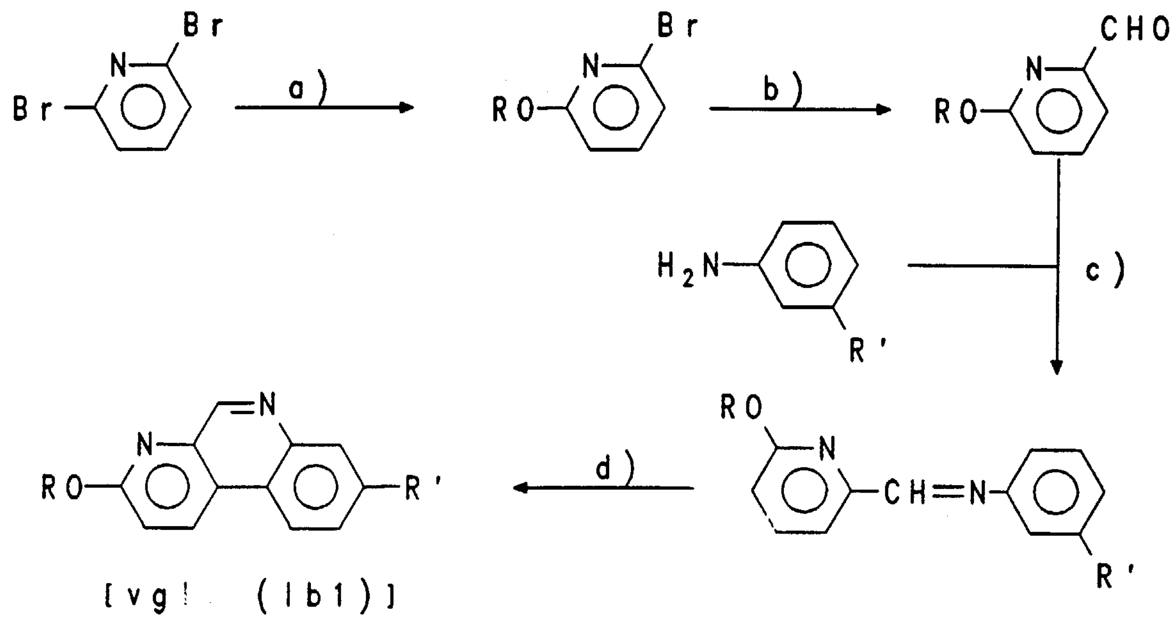


[vgl. (Ib1)]

a) NaOR in ROH
b) 1. BuLi; 2. DMF; 3. $H^+$; analog J. Org. Chem. 51, 3762 (1986)
c) z.B. analog Angew. Chemie 81, 903 (1969)
d) h·ν/$O_2$; z.B. analog J. Am. Chem. Soc. 84, 4361 (1962)

Schema 2

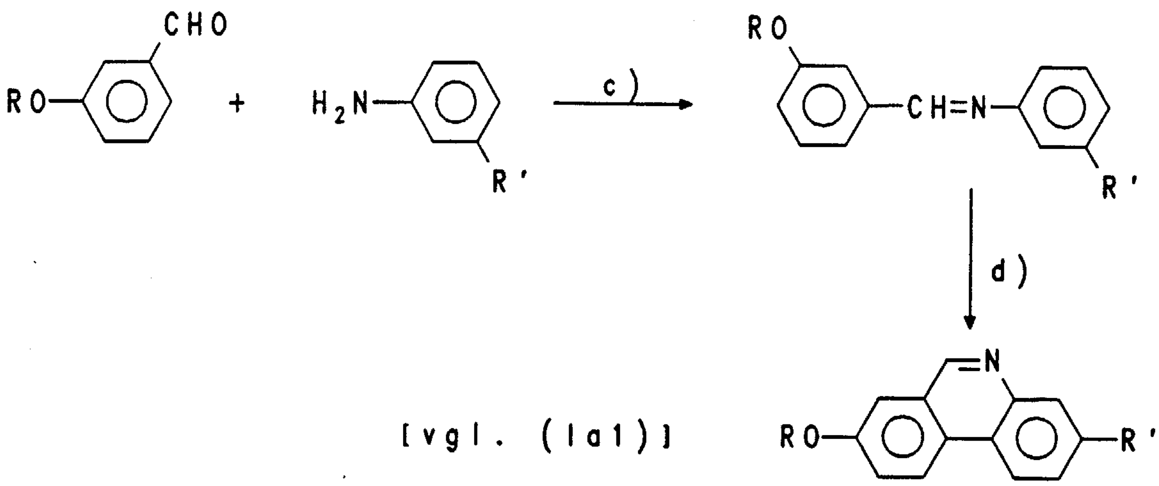


[vgl. (Ia1)]

Schema 3

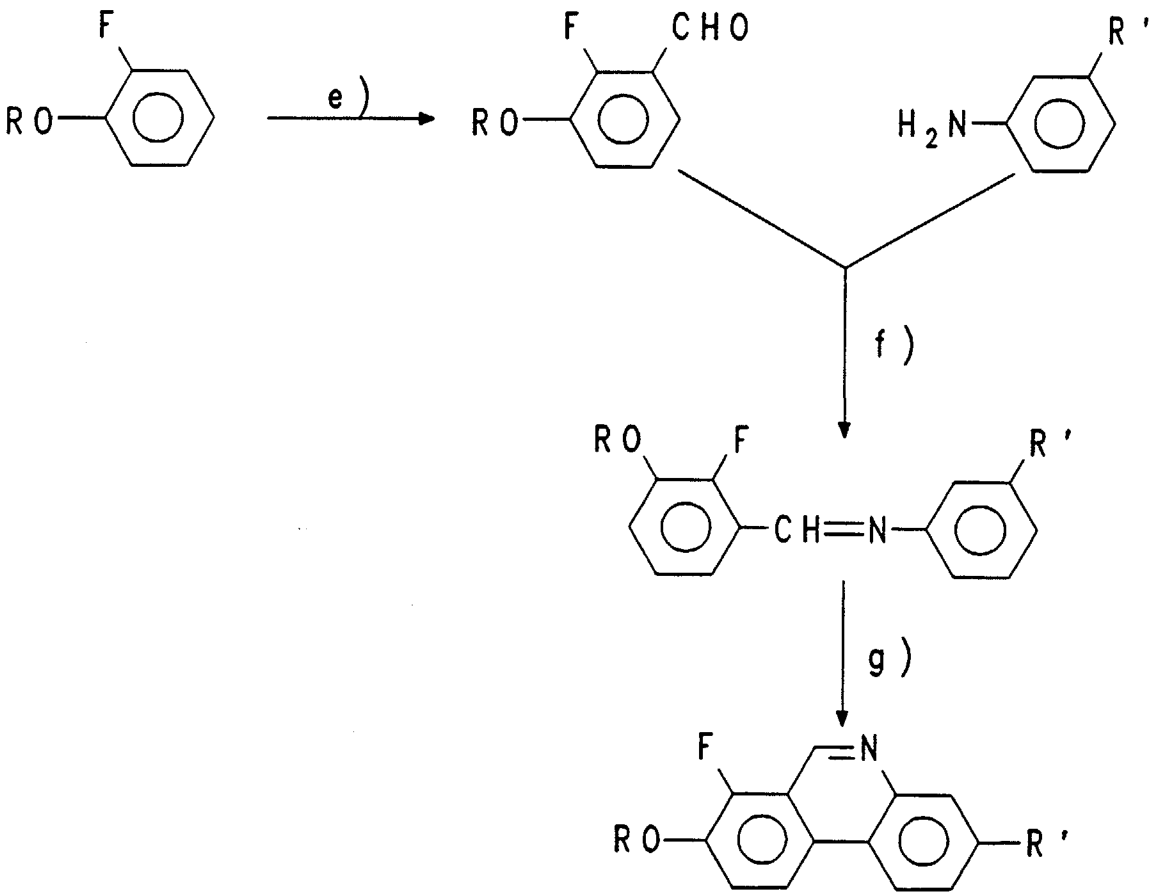


[vgl. (Ic1)]

Die Synthese des Restes $R^1$-$(A^1$-$M^1)_m$ bzw. $(M^2$-$A^2)_n$-$R^2$ oder geeigneter reaktiver Derivate davon oder auch anderer geeigneter Vorläufer dieser Gruppierung erfolgt nach dem Fachmann bekannten Methoden.

Für die Synthese spezieller aromatischer Kerne, Bestandteil der Verbindungen der Formeln (I) bzw. (Ia) wird verwiesen auf DE-A 23 44 732, 24 50 088, 24 29 093, 25 02 904, 26 36 684, 27 01 591 und 27 52 975 für Verbindungen mit 1,4-Cyclohexylen und 1,4-Phenylen-Gruppen; DE-A 26 41 724 für Verbindungen mit Pyrimidin-2,5-diyl-Gruppen; DE-A 40 26 223 und EP-A 03 91 203 für Verbindungen mit Pyridin-2,5-diyl-Gruppen; DE-A 32 31 462 für Verbindungen mit Pyridazin-3,6-diyl-Gruppen; N. Miyaura, T. Yanagi und A. Suzuki in Synthetic Communications 11 (1981), S. 513-519, DE-C- 3 930 663, M.J. Sharp, W. Cheng, V. Snieckus in Tetrahedron Letters 28 (1987), S. 5093 ff.; G.W. Gray in J. Chem. Soc. Perkin Trans II, 1989, S. 2041 ff. und Mol. Cryst. Liq. Cryst. 172 (1989), S. 165 ff., 204 (1991), S. 43 ff. und S. 91 ff.; EP-A O 449 015; WO 89/12039; WO 89/03821; EP-A 0 354 434 für die direkte Verknüpfung von Aromaten und Heteroaromaten; DE-A 32 01 721 für Verbindungen mit $-CH_2CH_2-$Brückengliedern und Koji Seto et al. in Liquid Crystals 8 (1990), S. 861-870 für Verbindungen mit -C≡C- Brückengliedern.

Die Darstellung disubstituierter Pyridine, disubstituierter Pyrazine, disubstituierter Pyrimidine und disubstituierter Pyridazine findet sich beispielsweise in den entsprechenden Bänden der Serie "The Chemistry of Heterocyclic Compounds" von A. Weissberger und E.C. Taylor (Herausgeber).

Dioxanderivate werden zweckmäßig durch Reaktion eines entsprechenden Aldehyds (oder eines seiner reaktionsfähigen Derivate) mit einem entsprechenden 1,3-Diol (oder einem seiner reaktionsfähigen Derivate) hergestellt, vorzugsweise in Gegenwart eines inerten Lösungsmittels, wie Benzol oder Toluol, und/oder eines Katalysators, z.B. einer starken Säure, wie Schwefelsäure, Benzol- oder p-Toluolsulfonsäure, bei Temperaturen zwischen etwa 20°C und etwa 150°C, vorzugsweise zwischen 80°C und 120°C. Als reaktionsfähige Derivate der Ausgangsstoffe eignen sich in erster Linie Acetale.

Die genannten Aldehyde und 1,3-Diole sowie ihre reaktionsfähigen Derivate sind zum Teil bekannt, zum Teil können sie ohne Schwierigkeiten nach Standardverfahren der Organischen Chemie aus literaturbekannten Verbindungen hergestellt werden. Beispielsweise sind die Aldehyde durch Oxydation entsprechender Alkohole oder durch Reduktion von Nitrilen oder entsprechenden Carbonsäuren oder ihrer Derivate, die Diole durch Reduktion entsprechender Diester erhältlich.

Verbindungen, worin ein aromatischer Ring durch mindestens ein F-Atom substituiert ist, können auch aus den entsprechenden Diazoniumsalzen durch Austausch der Diazoniumgruppe gegen ein Fluoratom, z.B. nach den Methoden von Balz und Schiemann, erhalten werden.

Was die Verknüpfung der Ringsysteme miteinander angeht, sei beispielsweise verwiesen auf:
N. Miyaura, T. Yanagai und A. Suzuki in Synthetic Communications 11 (1981), 513-519 DE-C-39 30 663, M.J. Sharp, W. Cheng, V. Snieckus in Tetrahedron Letters 28 (1987) 5093; G.W. Gray in J. Chem. Soc. Perkin Trans II 1989, 2041 und Mol. Cryst. Liq. Cryst. 172 (1989) 165, 204 (1991) 43 und 91; EP-A O 449 015; WO-A 89/12039; WO-A 89/03821; EP-A O 354 434 für die direkte Verknüpfung von Aromaten und Heteroaromaten; DE-A 32 01 721 für Verbindungen mit $-CH_2CH_2-$Brückengliedern und Koji Seto et al. in Liquid Crystals 8 (1990) 861-870 für Verbindungen mit -C≡C-Brückengliedern.

Ester der Formel (I) können auch durch Veresterung entsprechender Carbonsäuren (oder ihrer reaktionsfähigen Derivate) mit Alkoholen bzw. Phenolen (oder ihren reaktionsfähigen Derivaten) oder nach der DCCI-Methode (DCCI = Dicyclohexylcarbodiimid) erhalten werden.

Die entsprechenden Carbonsäuren und Alkohole bzw. Phenole sind bekannt und können in Analogie zu bekannten Verfahren hergestellt werden.

Als reaktionsfähige Derivate der genannten Carbonsäuren eignen sich insbesondere die Säurehalogenide, vor allem die Chloride und Bromide, ferner die Anhydride, z.B. auch gemischte Anhydride, Azide oder Ester, insbesondere Alkylester mit 1-4 C-Atomen in der Alkylgruppe.

Als reaktionsfähige Derivate der genannten Alkohole bzw. Phenole kommen insbesondere die entsprechenden Metallalkoholate bzw. Phenolate, vorzugsweise eines Alkalimetalls, wie Natrium oder Kalium, in Betracht.

Die Veresterung wird vorteilhaft in Gegenwart eines inerten Lösungsmittels durchgeführt. Gut geeignet sind insbesondere Ether, wie Diethylether, Di-n-butylether, THF, Dioxan oder Anisol, Ketone, wie Aceton, Butanon oder Cyclohexanon, Amide, wie DMF oder Phosphorsäurehexamethyltriamid, Kohlenwasserstoffe, wie Benzol, Toluol oder Xylol, Halogenkohlenwasserstoffe, wie Tetrachlorkohlenstoff, Dichlormethan oder Tetrachlorethylen und Sulfoxide, wie Dimethylsulfoxid oder Sulfolan.

Ether der Formel (I) sind durch Veretherung entsprechender Hydroxyverbindungen, vorzugsweise entsprechender Phenole, erhältlich, wobei die Hydroxyverbindung zweckmäßig zunächst in ein entsprechendes Metallderivat, z.B. durch Behandeln mit NaH, $NaNH_2$, NaOH, KOH, $Na_2CO_3$ oder $K_2CO_3$ in das entsprechende Alkalimetallalkoholat oder Alkalimetallphenolat übergeführt wird. Dieses kann dann mit dem entsprechenden Alkylhalogenid, Sulfonat oder Dialkylsulfat umgesetzt werden, zweckmäßig in einem inerten Lösungsmittel, wie Aceton, 1,2-Dimethoxyethan, DMF oder Dimethylsulfoxid, oder auch mit einem Überschuß an wäßriger oder wäßrig-alkoholischer NaOH oder KOH bei Temperaturen zwischen etwa 20° und 100°C.

Was die Synthese spezieller Reste R angeht, sei zusätzlich beispielsweise verwiesen auf EP-A O 355 008 für Verbindungen mit siliziumhaltigen Seitenketten und EP-A O 292 954 und EP-A O 398 155 für Verbindungen mit Cyclopropylgruppen in der Seitenkette.

Mit der Bereitstellung von ferroelektrischen Flüssigkristallmischungen, die Verbindungen der Formel (I) enthalten wird ganz allgemein die Palette der flüssigkristallinen Mischungen, die sich unter verschiedenen anwendungstechnischen Gesichtspunkten eignen, erheblich verbreitert.

In diesem Zusammenhang besitzen die Verbindungen der Formel (I) einen breiten Anwendungsbereich. In Abhängigkeit von der Auswahl der Substituenten können sie als Basismaterialien dienen, aus denen die erfindungsgemäßen flüssigkristalline Phasen zum überwiegenden Teil zusammengesetzt sind; es können aber auch Verbindungen der Formel (I) flüssigkristallinen Basismaterialien aus anderen Verbindungsklassen zugesetzt werden, um beispielsweise die dielektrische und/oder optische Anisotropie eines erfindungsgemäßen Dielektrikums zu beeinflussen und/oder um dessen Schwellenspannung und/oder dessen Viskosität zu optimieren. Besonders geeignet sind die Verbindungen der Formel (I), um schon in geringen Zumischmengen die dielektrische Anistropie, $\Delta\varepsilon$, in Richtung auf höhere negative Werte zu beeinflussen.

Die erfindungsgemäßen Flüssigkristallmischungen enthalten im allgemeinen 2 bis 35, vorzugsweise 2 bis 25, besonders bevorzugt 2 bis 20 Komponenten.

Sie enthalten im allgemeinen 0,01 bis 80 Gew.-%, vorzugsweise 0,1 bis 60 Gew.-%, besonders bevorzugt 0,1 bis 30 Gew.-%, an einer oder mehreren, vorzugsweise 1 bis 10, besonders bevorzugt 1 bis 5, ganz besonders bevorzugt 1 bis 3, der erfindungsgemäßen Verbindungen der Formel (I).

Weitere Komponenten von erfindungsgemäßen Flüssigkristallmischungen werden vorzugsweise ausgewählt aus den bekannten Verbindungen mit smektischen und/oder nematischen und/oder cholesterischen und/oder antiferroelektrischen Phasen. Dazu gehören z.B.:

- Derivate des Phenylpyrimidins, wie beispielsweise in WO 86/06401, US-A 4 874 542 beschrieben,
- metasubstituierte Sechsringaromaten, wie beispielsweise in EP-A-0 578 054 beschrieben,
- Siliziumverbindungen, wie beispielsweise in EP-A O 355 008 beschrieben,
- mesogene Verbindungen mit nur einer Seitenkette, wie in EP-A 0 541 081 beschrieben,
- Hydrochinonderivate, wie beispielsweise in EP-A-O 603 786 beschrieben,
- Pyridylpyrimidine, wie beispielsweise in WO 92/12974 beschrieben,
- Phenylbenzoate, wie beispielsweise bei P. Keller, Ferroelectrics 58 (1984), 3 und J. W. Goodby et al., Liquid Crystals and Ordered Fluids, Bd. 4, New York 1984 beschrieben und
- Thiadiazo!e, wie z.B. in EP-B 309 514 beschrieben.

Als chirale, nicht racemische Dotierstoffe kommen beispielsweise in Frage:

- optisch aktive Phenylbenzoate, wie beispielsweise bei P. Keller, Ferroelectrics 1984, 58, 3 und J. W. Goodby et al., Liquid Crystals and Ordered Fluids, Bd. 4, New York 1984 beschrieben,
- optisch aktive Oxiranether, wie beispielsweise in EP-A 0 263 437 und WO-A 93/13093 beschrieben,
- optisch aktive Oxiranester, wie beispielsweise in EP-A 0 292 954 beschrieben,
- optisch aktive Dioxolanether, wie beispielsweise in EP-A 0 351 746 beschrieben,
- optisch aktive Dioxolanester, wie beispielsweise in EP-A 0 361 272 beschrieben, und
- optisch aktive Tetrahydrofuran-2-carbonsäureester, wie beispielsweise in EP-A 0 355 561 beschrieben.

Die erfindungsgemäßen Mischungen wiederum können Anwendung finden in elektrooptischen oder vollständig optischen Elementen, z.B. Anzeigeelementen, Schaltelementen, Lichtmodulatoren, Elementen zur Bildbearbeitung und/oder Signalverarbeitung oder allgemein im Bereich der nichtlinearen Optik.

Erfindungsgemäße flüssigkristalline Mischungen, die Verbindungen der allgemeinen Formel (I) enthalten, sind besonders für die Verwendung in elektrooptischen Schalt- und Anzeigevorrichtungen (Displays) geeignet. Diese Displays sind üblicherweise so aufgebaut, daß eine Flüssigkristallschicht beiderseitig von Schichten eingeschlossen ist, die üblicherweise, in dieser Reihenfolge ausgehend von der LC-Schicht, mindestens eine Orientierungsschicht, Elektroden und eine Begrenzungsscheibe (z.B. aus Glas) sind. Darüberhinaus enthalten sie Abstandshalter, Kleberahmen, Polarisatoren sowie für Farbdisplays dünne Farbfilterschichten. Weitere mögliche Komponenten sind Antireflex-, Passivierungs-, Ausgleichs- und Sperrschichten sowie elektrisch-nichtlineare Elemente, wie Dünnschichttransistoren (TFT) und Metall-Isolator-Metall-(MIM)-Elemente. Im Detail ist der Aufbau von Flüssigkristalldisplays bereits in einschlägigen Monographien beschrieben (siehe z.B. E. Kaneko, "Liquid Crystal TV Displays: Principles and Applications of Liquid Crystal Displays", KTK Scientific Publishers 1987).

Ferner sind die erfindungsgemäßen Mischungen für Feldbehandlung, d. h. zum Betrieb in der Quasi-Bookshelf-Geometrie (QBG), (siehe z. B. H. Rieger et al., SID 91 Digest (Anaheim) 1991, p. 396), geeignet.

Ebenso sind die erfindungsgemäßen Mischungen geeignet für die Verwendung in ferroelektrischen Flüssigkristallanzeigen, die auf Nutzung des DHF-Effekts oder des PSFLCD-Effekts (Pitch Stabilized Ferroelectric Liquid Crystal Display, auch SBF = Short Pitch Bistable Ferroelectric Effect genannt) beruhen.

Die vorliegende Erfindung wird an Hand der folgenden Beispiele näher erläutert, ohne sie dadurch einschränken zu wollen.

Beispiel 1

4-Fluor-8-methoxy-3-octyloxy-phenanthridin

Eine Lösung von 2,5 g 2-Fluor-3-octyloxy-benzaldehyd (herstellt durch Umsetzung von 2-Octyloxy-fluorbenzol mit Lithiumdiisopropylamid und nachfolgende Reaktion mit N,N-Dimethylformamid) in 50 ml Toluol wird bei 110°C 30 min mit 1,4 g 3-Methoxy-anilin erhitzt. Nach Abdestillation des Lösungsmittels im Vakuum am Rotationsverdampfer wird rohes (2-Fluor-3-octyloxy)benzyliden-(3-methoxy)anilin erhalten. Dieses wird, gelöst in Cyclohexan unter Zusatz von 4 Mol-% Iod, für 8 Stunden bei 25°C in einer Quarz-Apparatur UV-Licht ausgesetzt. Nach Chromatographie an $SiO_2$ mit Dichlormethan und Umkristallisation aus Acetonitril werden 0,8 g 4-Fluor-8-methoxy-3-octyloxy-phenanthridin erhalten.

Analog Beispiel 1 werden erhalten:

Bsp. 2 8-Decyloxy-4-fluor-3-octyloxy-phenanthridin
Bsp. 3 4,7-Difluoro-3,8-bis(octyloxy)-phenanthridin
Bsp. 4 7-Fluor-3,8-bis(hexyloxy)-phenanthridin
Bsp. 5 8-Methoxy-3-octyloxy-4-aza-phenanthridin
Bsp. 6 8-Heptyloxy-3-octyloxy-4-aza-phenanthridin
Bsp. 7 7-Fluor-3,8-bis(octyloxy)-4-aza-phenanthridin
Bsp. 8 7-Fluor-3-(4-butyl-dimethylsilyl)butyloxy-8-octyloxy-phenanthridin
Bsp. 9 4-Fluor-3-octyloxy-phenanthridin

Anwendungsbeispiel 1

Eine ferroelektrische Flüssigkristallmischung, bestehend aus

Gewichtsanteile

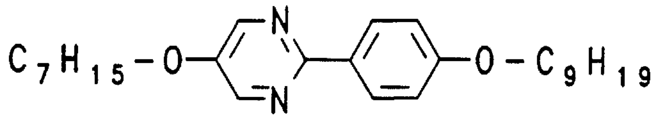

3,77 %

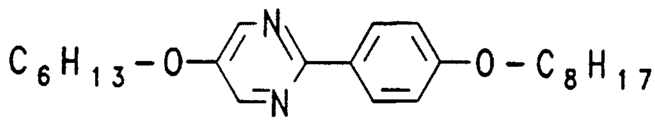

4,25 %

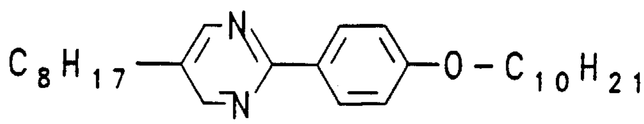

4,70 %

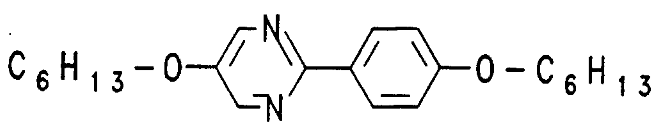

3,95 %

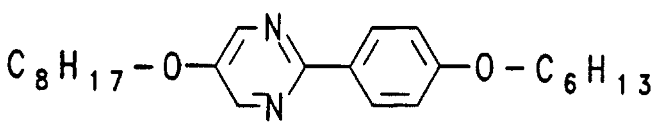

4,25 %

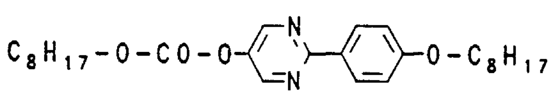

8,00 %

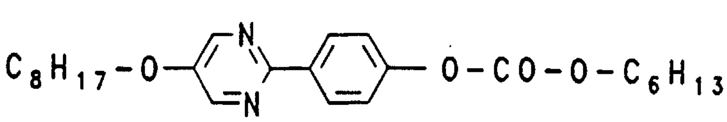

7,47 %

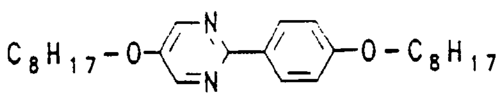

4,56 %

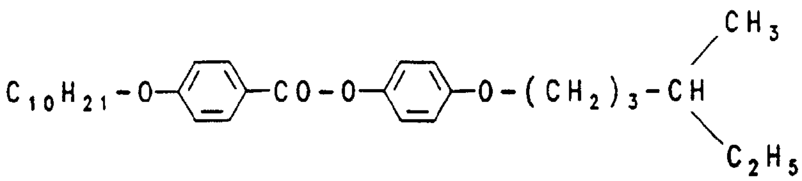

8,11 %

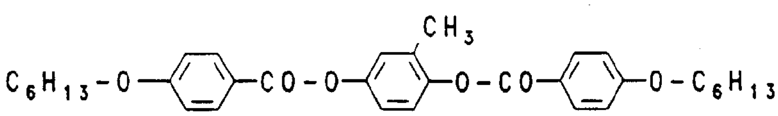

2,30 %

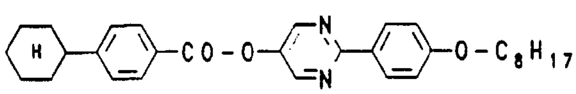

3,22 %

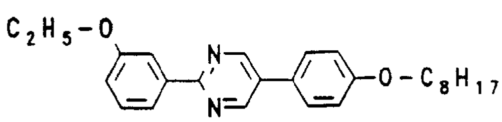

6,36 %

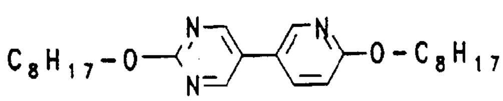

3,61 %

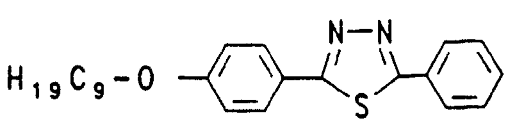

5,92 %

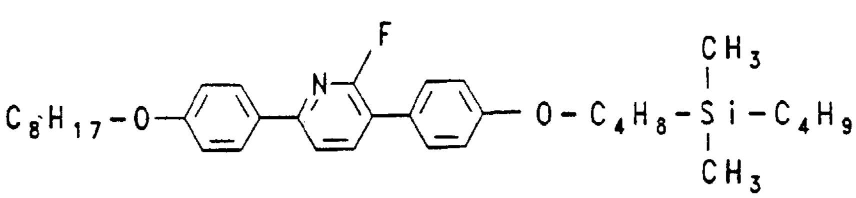

7,32 %

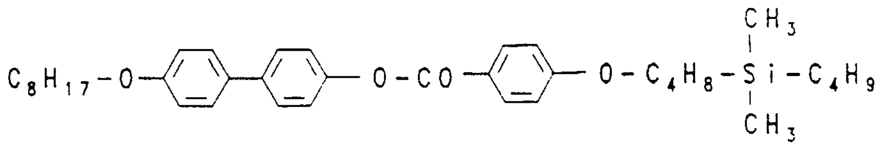

8,06%

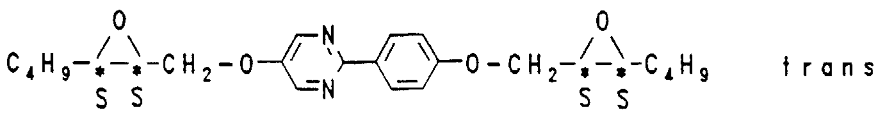

2,73 %

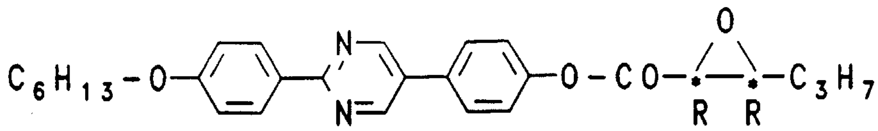

6,71%

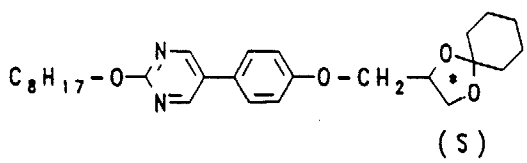

2,00%

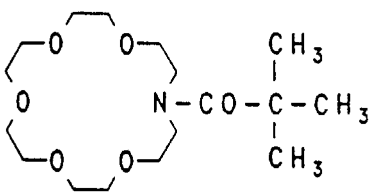

0,65 %

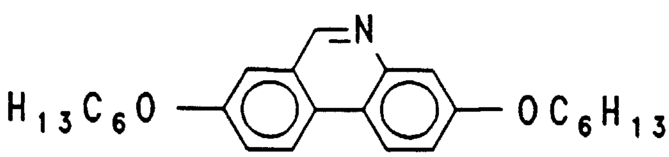

2,14 %

zeigt folgende flüssigkristalline Phasenbereiche:

X -29 $S_C^*$ 74 $S_A$ 86 N* 95 I Die spontane Polarisation beträgt bei 25°C 40 n $C/cm^2$.

Anwendungsbeispiel 2

Eine ferroelektrische Flüssigkristallmischung, bestehend aus

| | |
|---|---|
| 4,6 Gew.-% | 2-(4-Ethoxyphenyl)-5-octyloxy-pyrimidin |
| 12,6 Gew.-% | 2-(4-Butoxyphenyl)-5-octyloxy-pyrimidin |
| 13,9 Gew.-% | 2-(4-Hexyloxyphenyl)-5-octyloxy-pyrimidin |

(fortgesetzt)

| | |
|---|---|
| 6,4 Gew.-% | 9-Octyloxy-2-(4-octyloxyphenyl)-pyrimidin |
| 26,9 Gew.-% | 2-[4-(10-Undecen-1-yl)oxyphenyl]-5-octyl-pyrimidin |
| 11,4 Gew.-% | trans-4-Pentylcyclohexancarbonsäure-[4-(5-octyloxy-pyrimidin-2-yl)]phenyl-ester |
| 10,7 Gew.-% | 4-[2-((S)-7-Methyl-monyloxy)pyrimidin-5-yl]phenyl-(2S, 3S)-2-chloro-3-methyl-pentansäureester |
| 1,0 Gew.-% | 5-[4-((S)-3,7-Dimethyl-oct-6-en-1-yl)oxyphenyl]-2-octyloxy-pyrimidin |

wird mit 1,95 Gew.-% 4-Fluor-6-hexyloxy-3-octyloxy-phenantheridin versetzt; der Übergang $X/S_C^*$ steigt von -1 auf 0°C, der Übergang $S_C^*/S_A$ fällt von 71 auf 69°C, der Übergang $S_A/N^*$ bleibt unverändert bei 79°C, während $\Delta\varepsilon$ (20 $KH_2$) sich von -0,8 auf -1,0 verändert.

Diese Beispiel belegt, daß schon mit geringen Mengen der erfindungsgemäßen Verbindungen bei guter Mesophenenkompatibilität die dielektrische Anisotropie "maßgeschneidert" werden kann.

**Patentansprüche**

**1.** Ferroelektrische Flüssigkristallmischung, enthaltend ein oder mehrere Phenanthridinderivate der Formel (I),

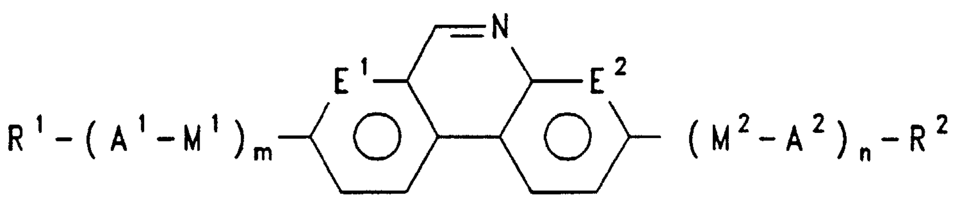

(I)

in der die Symbole und Indizes folgende Bedeutungen haben:

$E^1$, $E^2$ sind gleich oder verschieden -N-, -CF- oder -CH-;

$R^1$, $R^2$ sind gleich oder verschieden ein geradkettiger oder verzweigter Alkylrest mit 1 bis 20 Kohlenstoffatomen (mit oder ohne asymmetrisches Kohlenstoffatom), wobei auch eine oder mehrere $CH_2$-Gruppen durch -O-, -S-, -C≡C-, Cyclopropan-1,2-diyl oder $-Si(CH_3)_2-$ ersetzt sein können, mit der Maßgabe, daß Sauerstoff und Schwefelatome nicht unmittelbar miteinander gebunden sein dürfen, und wobei auch ein oder mehrere Wasserstoffatome des Alkylrests durch Fluor ersetzt sein können, oder eine der nachfolgenden chiralen Gruppen (optisch aktiv oder racemisch):

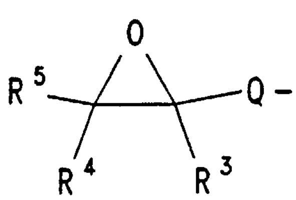

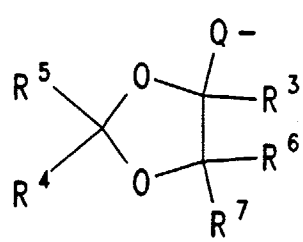

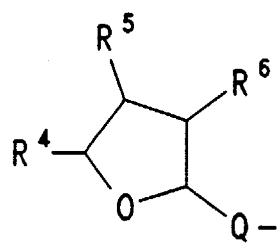

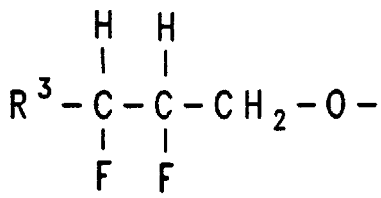
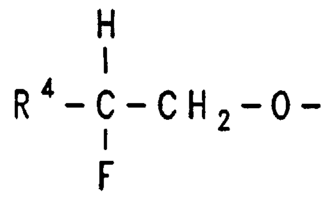

eine der beiden Gruppen $R^1$, $R^2$ kann auch Wasserstoff sein;

| | |
|---|---|
| $R^3$, $R^4$, $R^5$, $R^6$, $R^7$ | sind gleich oder verschieden Wasserstoff oder ein geradkettiger oder verzweigter Alkylrest mit 1 bis 16 Kohlenstoffatomen (mit oder ohne asymmetrisches Kohlenstoffatom), wobei auch eine $CH_2$-Gruppe durch -O- ersetzt sein kann und wobei ein oder mehrere Wasserstoffatome des Alkylrests durch Fluor ersetzt sein können, $R^4$ und $R^5$ können zusammen auch $-(CH_2)_4-$ oder $-(CH_2)_5-$ bedeuten, wenn sie an ein Dioxolansystem gebunden sind; |
| Q | ist $-CH_2-O-$, -CO-O- oder eine Einfachbindung; |
| $M^1$, $M^2$ | sind gleich oder verschieden -CO-O-, -O-CO-, $-CH_2-O-$, $-O-CH_2-$, $-CH_2-CH_2-$, $-C{\equiv}C-$ oder eine Einfachbindung; |
| $A^1$, $A^2$ | sind gleich oder verschieden 1,4-Phenylen, wobei ein oder mehrere H-Atome durch F ersetzt sein können, trans-1,4-Cyclohexylen, Pyrimidin-2,5-diyl, Pyridin-2,5-diyl, wobei ein H-Atom durch F ersetzt sein kann, (1,3,4)-Thiadiazol-2,5-diyl, 1,3-Dioxan-2,5-diyl, 1,3-Dithian-2,5-diyl, 1,3-Thiazol-2,4-diyl, 1,3-Thiazol-2,5-diyl, Thiophen-2,4-diyl oder Thiophen-2,5-diyl; |
| m, n | sind Null oder Eins, in der Summe jedoch maximal Eins. |

**2.** Ferroelektrische Flüssigkristallmischung nach Anspruch 1, dadurch gekennzeichnet, daß sie eine Verbindung der Formel (I) enthält, in der die Gruppe

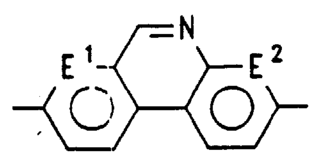

eine der folgenden Bedeutungen hat:

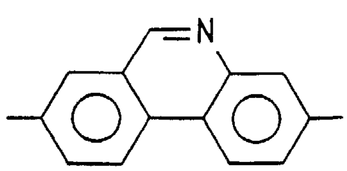
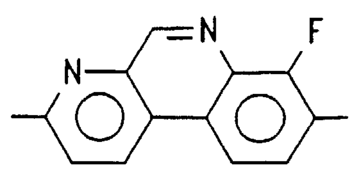
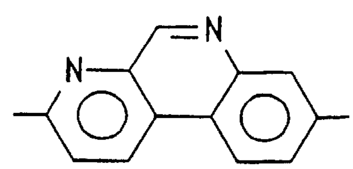

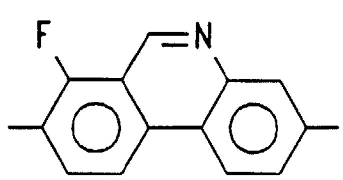
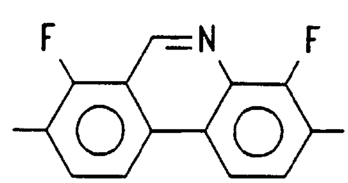

3. Ferroelektrische Flüssigkristallmischung nach Anspruch 1 oder 2, dadurch gekennzeichnet, daß in der Verbindung der Formel (I) $R^1$, $R^2$ geradkettige oder verzweigte Alkylreste (mit oder ohne asymmetrisches C-Atom) mit 1 bis 16 C-Atomen sind, wobei auch eine oder mehrere $CH_2$-Gruppen durch -O-, Cyclopropan-1,2-diyl oder $-Si(CH_3)_2-$ ersetzt sein können mit der Maßgabe, daß Sauerstoffatome nicht unmittelbar verbunden sein dürfen, und/oder wobei ein oder mehrere H-Atome der Alkylreste auch durch F substituiert sein können.

4. Ferroelektrische Flüssigkristallmischung nach einem oder mehreren der Ansprüche 1 bis 3, dadurch gekennzeichnet, daß sie eine Verbindung der Formel (I) aus der folgenden Gruppe enthält, wobei $R^1$ und $R^2$ geradkettige oder verzweigte Alkyloxyreste mit 1 bis 10 C-Atomen sind, wobei auch eine durch mindestens zwei weitere $-CH_2$-Gruppen vom Kern getrennte $-CH_2$-Gruppe durch $-Si(CH_3)_2$-ersetzt sein kann:

a) 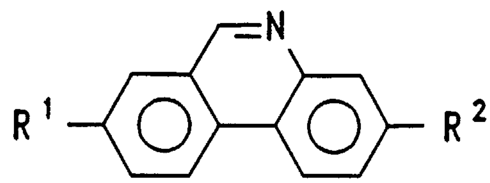

b) 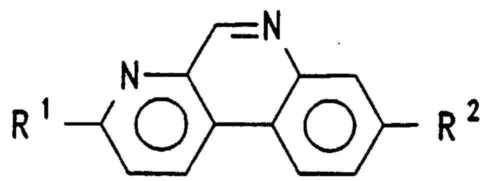

c) 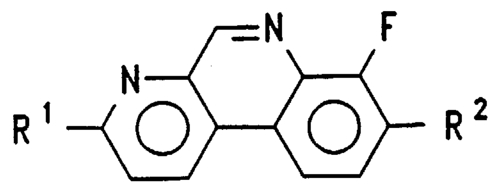

d) 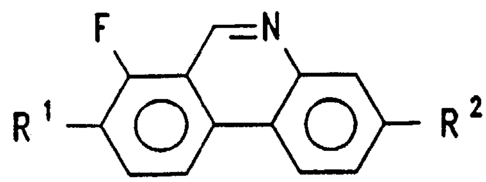

e) 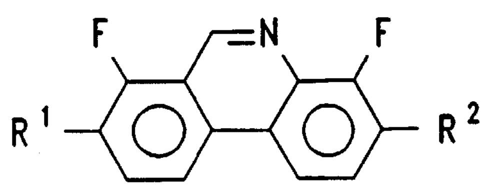

5. Ferroelektrische Flüssigkristallmischung nach einem oder mehreren der Ansprüche 1 bis 4, dadurch gekennzeichnet, daß sie aus 2 bis 20 Komponenten besteht und 1 bis 10 Verbindungen der Formel (I) enthält.

6. Ferroelektrische Flüssigkristallmischung nach einem oder mehreren der Ansprüche 1 bis 5, dadurch gekennzeichnet, daß sie 0,01 bis 80 Gew.-% an einer oder mehreren Verbindungen der Formel (I) enhält.

7. Verwendung einer ferroelektrischen Flüssigkristallmischung nach einem oder mehreren der Ansprüche 1 bis 6 in elektrooptischen Schalt- und/oder Anzeigeelementen.

8. Elektrooptisches Schalt- und/oder Anzeigeelement, dadurch gekennzeichnet, daß es eine ferroelektrische Flüssigkristallmischung nach einem oder mehreren der Ansprüche 1 bis 6 enthält.

9. Phenanthridinderivate der Formel (Ia),

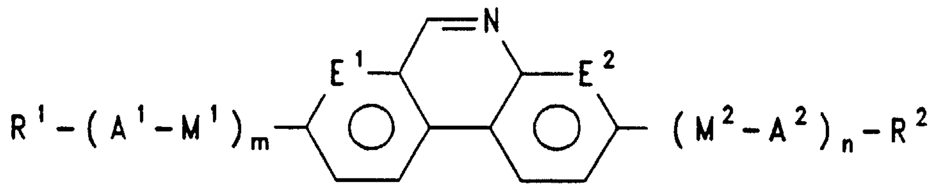

(Ia)

in der die Symbole und Indizes folgende Bedeutungen haben:

$E^1$, $E^2$ sind gleich oder verschieden -N-, -CF- oder -CH-;

$R^1$, $R^2$ sind gleich oder verschieden ein geradkettiger oder verzweigter Alkylrest mit 1 bis 20 Kohlenstoffatomen (mit oder ohne asymmetrisches Kohlenstoffatom), wobei auch eine oder mehrere $CH_2$-Gruppen durch -O-, -S-, -C≡C-, Cyclopropan-1,2-diyl oder $-Si(CH_3)_2-$ ersetzt sein können, mit der Maßgabe, daß Sauerstoff und Schwefelatome nicht unmittelbar miteinander gebunden sein dürfen, und wobei auch ein oder mehrere Wasserstoffatome des Alkylrests durch Fluor ersetzt sein können, oder eine der nachfolgenden chiralen Gruppen (optisch aktiv oder racemisch):

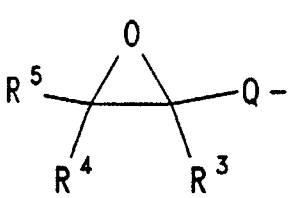

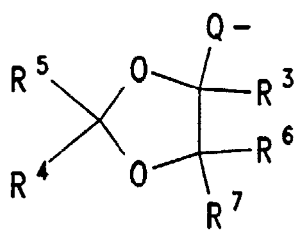

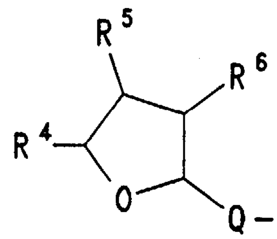

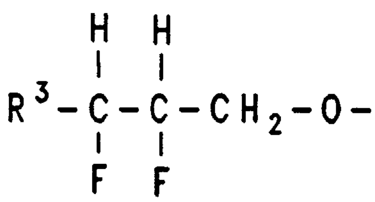

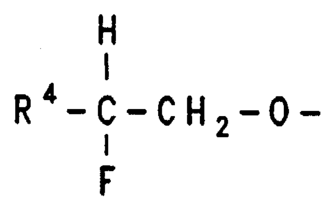

$R^3$, $R^4$, $R^5$, $R^6$, $R^7$ sind gleich oder verschieden Wasserstoff oder ein geradkettiger oder verzweigter Alkylrest mit 1 bis 16 Kohlenstoffatomen (mit oder ohne asymmetrisches Kohlenstoffatom, wobei auch eine $CH_2$-Gruppe durch -O- ersetzt sein kann und wobei ein oder mehrere Wasserstoffatome des Alkylrests durch Fluor ersetzt sein können, $R^4$ und $R^5$ können zusammen auch $-(CH_2)_4-$ oder $-(CH_2)_5-$ bedeuten, wenn sie an ein Dioxolansystem gebunden sind;

Q ist $-CH_2-O-$, -CO-O- oder eine Einfachbindung;

$M^1$, $M^2$ sind gleich oder verschieden -CO-O-, -O-CO-, $-CH_2-O-$, $-O-CH_2-$, $-CH_2-CH_2-$, -C≡C- oder eine Einfachbindung;

$A^1$, $A^2$ sind gleich oder verschieden 1,4-Phenylen, wobei ein oder mehrere H-Atome durch F ersetzt sein können, trans-1,4-Cyclohexylen, Pyrimidin-2,5-diyl, Pyridin-2,5-diyl, wobei ein H-Atom durch F ersetzt sein kann, (1,3,4)-Thiadiazol-2,5-diyl, 1,3-Dioxan-2,5-diyl, 1,3-Dithian-2,5-diyl, 1,3-Thiazol-2,4-diyl, 1,3-Thiazol-2,5-diyl, Thiophen-2,4-diyl oder Thiophen-2,5-diyl;

m, n sind Null oder Eins, in der Summe jedoch maximal Eins.

## Claims

**1.** A ferroelectric liquid-crystal mixture containing one or more phenanthridine derivatives of the formula (I)

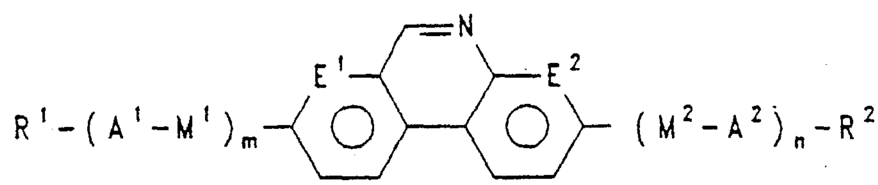

(I)

in which the symbols and indices have the following meanings:

$E^1$ and $E^2$ are identical or different and are -N-, -CF- or -CH-;

$R^1$ and $R^2$ are identical or different and. are a straight-chain or branched alkyl radical having 1 to 20 carbon atoms (with or without an asymmetrical carbon atom), where one or more $CH_2$ groups may also be replaced by -O-, -S-, -C≡C-, cyclo-propane-1,2-diyl or $-Si(CH_3)_2-$, with the proviso that oxygen and sulfur atoms must not be bonded directly to one another, and. where one or more hydrogen atoms of the alkyl radical may also be replaced by fluorine, or are one of the following chiral groups (optically active or racemic):

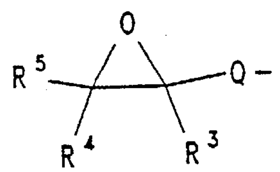

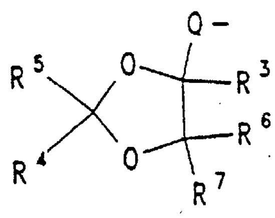

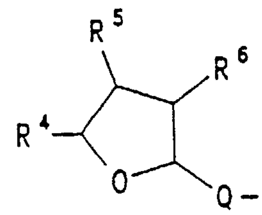

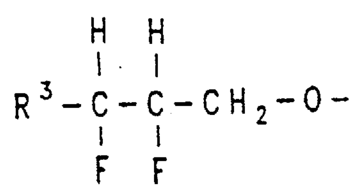

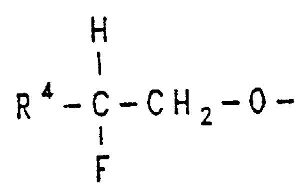

or one of the two groups $R^1$ and $R^2$ can alternatively be hydrogen;

$R^3$, $R^4$, $R^5$, $R^6$ and $R^7$ are identical or different and are hydrogen or a straight-chain or branched alkyl radical

having 1 to 16 carbon atoms (with or without an asymmetrical carbon atom); where one $CH_2$ group may also be replaced by -O- and where one or more hydrogen atoms of the alkyl radical may be replaced by fluorine; $R^4$ and $R^5$ may also together be -$(CH_2)_4$- or -$(CH_2)_5$- if they are bonded to a dioxolane system;

Q is -$CH_2$-O-, -CO-O- or a single bond;

$M^1$ and $M^2$ are identical or different and are -CO-O-, -O-CO-, -$CH_2$-O-, -O-$CH_2$-, -$CH_2$-$CH_2$-, -C≡C- or a single bond;

$A^1$ and $A^2$ are identical or different and are 1,4-phenylene, where one or more H atoms may be replaced by F, trans-1,4-cyclo-hexylene, pyrimidine-2,5-diyl, pyridine-2,5-diyl, where one H atom may be replaced by F, 1,3,4-thiadiazole-2,5-diyl, 1,3-dioxane-2,5-diyl, 1,3-dithiane-2,5-diyl, 1,3-thiazole-2,4-diyl, 1,3-thiazole-2, 5-diyl, thiophene-2,4-diyl or thiophene-2,5-diyl;

m and n are zero or one, but their sum is at most one.

**2.** A ferroelectric liquid-crystal mixture as claimed in claim 1, which contains a compound of the formula (I) in which the group

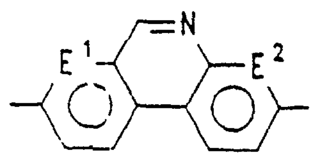

has one of the following meanings:

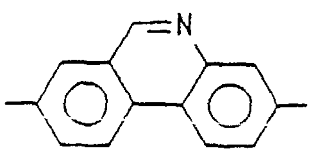
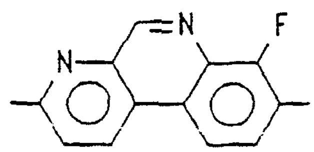
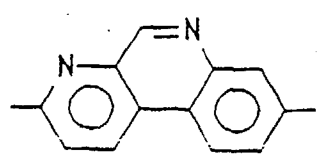

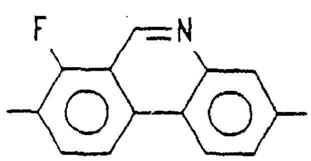
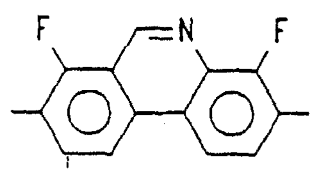

**3.** A ferroelectric liquid-crystal mixture as claimed in. claim 1 or 2, wherein $R^1$ and $R^2$ in the compound of the formula (I) are straight-chain or branched alkyl radicals (with or without an asymmetrical carbon atom) having 1 to 16 carbon atoms, where one or more $CH_2$ groups may also be replaced by -O-, cyclopropane-1,2-diyl or -$Si(CH_3)_2$-, with the proviso that oxygen atoms must not be bonded directly to one another, and/or where one or more H atoms of the alkyl radicals may also be replaced by F.

**4.** A ferroelectric liquid-crystal mixture as claimed in one or more of claims 1 to 3, which contains a compound of the formula (I) from the following group, where $R^1$ and $R^2$ are straight-chain or branched alkoxy radicals having 1 to 10 carbon atoms, where a -$CH_2$- group which is separated from the ring by at least two further -$CH_2$- groups may also be replaced by -$Si(CH_3)_2$-:

a ) 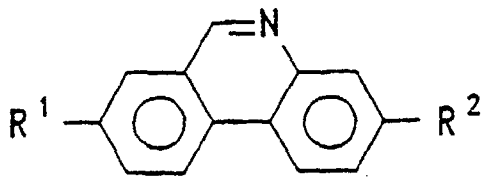

b ) 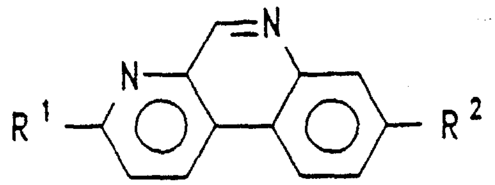

c ) 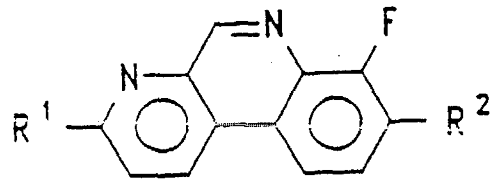

d ) 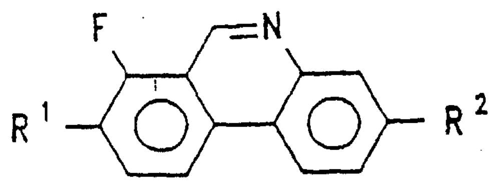

e ) 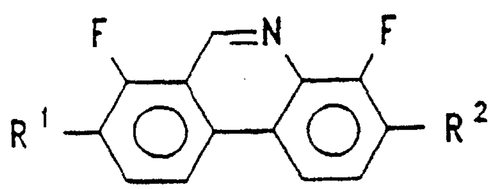

**5.** A ferroelectric liquid-crystal mixture as claimed in one or more of claims 1 to 4, which comprises 2 to 20 components and contains 1 to 10 compounds of the formula (I).

**6.** A ferroelectric liquid-crystal mixture as claimed in one or more of claims 1 to 5, which contains from 0.01 to 80% by weight of one or more compounds of the formula (I).

**7.** Use of a ferroelectric liquid-crystal mixture as claimed in one or more of claims 1 to 6 in electro-optical switching and/or display elements.

**8.** An electro-optical switching and/or display element which contains a ferroelectric liquid-crystal mixture as claimed in one or more of claims 1 to 6.

**9.** A phenanthridine derivative of the formula (Ia)

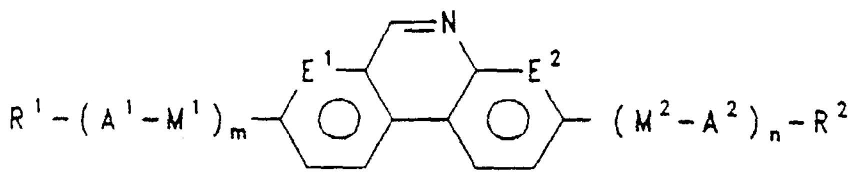

( I a )

in which the symbols and indices have the following meanings:

| | |
|---|---|
| $E^1$ and $E^2$ | are identical or different and are -N-, -CF- or -CH-; |
| $R^1$ and $R^2$ | are identical or different and are a straight-chain or branched alkyl radical having 1 to 20 carbon atoms (with or without an asymmetrical carbon atom), where one or more $CH_2$ groups may also be replaced by -O-, -S-, -C≡C-, cyclo-propane-1,2-diyl or $-Si(CH_3)_2-$, with the proviso that oxygen and sulfur atoms must not be bonded directly to one another, and where one or more hydrogen atoms of the alkyl radical may also be replaced by fluorine, or are one of the following chiral groups (optically active or racemic): |

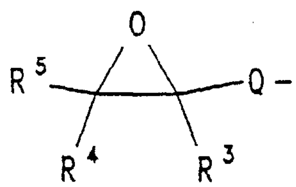
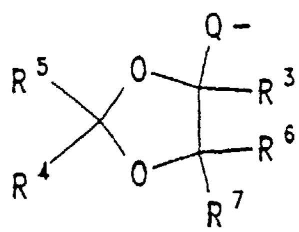
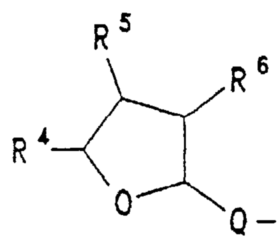

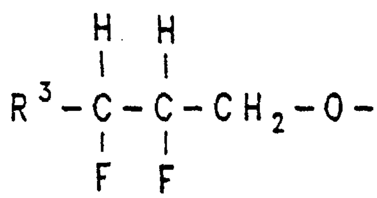
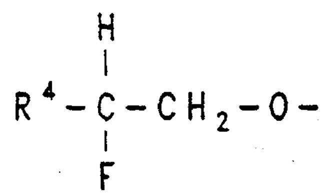

| | |
|---|---|
| $R^3$, $R^4$, $R^5$, $R^6$ and $R^7$ | are identical or different and are hydrogen or a straight-chain or branched alkyl radical having 1 to 16 carbon atoms (with or without an asymmetrical carbon atom), where one $CH_2$ group may also be replaced by -0- and where one or more hydrogen atoms of the alkyl radical may be replaced by fluorine; $R^4$ and $R^5$ may also together be $-(CH_2)_4-$ or $-(CR_2)_5-$, if they are bonded to a dioxolane system; |
| Q | is $-CH_2-O-$, -CO-O- or a single bond; |
| $M^1$ and $M^2$ | are identical or different and are -CO-O-, -O-CO-, $-CH_2-O-$, $-O-CH_2-$, $-CH_2-CH_2-$, -C≡C- or a single bond; |
| $A^1$ and $A^2$ | are identical or different and are 1,4-phenylene, where one or more H atoms may be replaced by F, trans-1,4-cyclohexylene, pyrimidine-2,5-diyl, pyridine-2,5-diyl, where one H atom may be replaced by F, 1,3,4-thiadiazole-2,5-diyl, 1,3-dioxane-2,5-diyl, 1,3-dithiane-2,5-diyl, 1,3-thiazole-2,4-diyl, 1,3-thiazole-2,5-diyl, thiophene-2,4-diyl or thiophene-2,5-diyl; |

m and n are zero or one, but their sum is at most one.

## Revendications

1. Mélange cristallin liquide ferroélectrique, contenant un ou plusieurs dérivés de phénanthridine de formule (I)

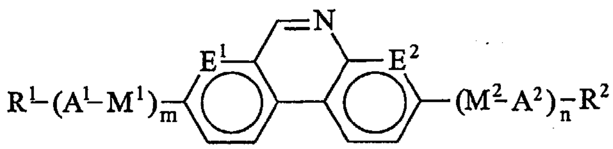

(I)

dans laquelle les symboles et les indices ont la signification suivante:

$E^1$ et $E^2$ sont identiques ou différents et représentent -N-, -CF- ou -CH-,

$R^1$ et $R^2$ sont identiques ou différents et représentent un reste alkyle linéaire ou ramifié de 1 à 20 atomes de carbone (avec ou sans atome de carbone asymétrique), dans lequel un ou plusieurs groupes $CH_2$ peuvent être remplacés par -O-, -S-, -C≡C-, cyclopropane-1,2-diyle ou $-Si(CH_3)_2-$, à condition que des atomes d'oxygène et de soufre ne soient pas directement liés entre eux, et dans lequel un ou plusieurs atomes d'hydrogène du reste alkyle peuvent être remplacés par du fluor, ou l'un des groupes chiraux suivants (optiquement actifs ou racémiques):

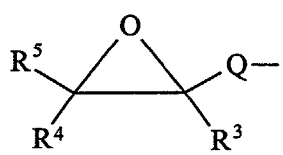

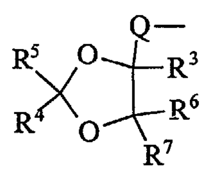

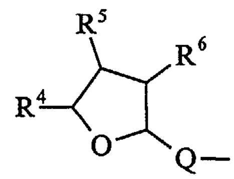

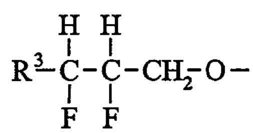

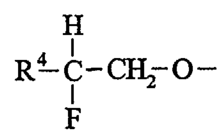 ,

l'un des deux groupes $R^1$ et $R^2$ pouvant aussi être un atome d'hydrogène;

$R^3$, $R^4$, $R^5$, $R^6$ et $R^7$ sont identiques ou différents et représentent un atome d'hydrogène ou un reste alkyle linéaire ou ramifié de 1 à 16 atomes de carbone (avec ou sans atome de carbone asymétrique) dans lequel un groupe $CH_2$ peut être remplacé par -O- et un ou plusieurs atomes d'hydrogène du groupe alkyle peuvent être remplacés par du fluor, $R^4$ et $R^5$ pouvant aussi former ensemble un groupe $-(CH_2)_4-$ ou $-(CH_2)_5-$ lorsqu'ils sont liés à un système dioxolane;

Q est $-CH_2-O-$, -CO-O- ou une liaison simple;

$M^1$ et $M^2$ sont identiques ou différents et représentent -CO-O-, -O-CO-, $-CH_2-O-$, $-O-CH_2-$, $-CH_2-CH_2-$, -C≡C- ou une liaison simple;

$A^1$ et $A^2$ sont identiques ou différents et représentent un groupe 1,4-phénylène dont un ou plusieurs atomes d'hydrogène peuvent être remplacés par F, trans-1,4-cyclohexylène, pyrimidine-2,5-diyle, pyridine-2,5-diyle, dont un atome d'hydrogène peut être remplacé par F, (1,3,4)-thiadiazole-2,5-diyle, 1,3-dioxane-2,5-diyle, 1,3-dithiane-2,5-diyle, 1,3-thiazole-2,4-diyle, 1,3-thiazole-2,5-diyle, thiophène-2,4-diyle ou thiophène-2,5-diyle;

m et n sont égaux à 0 ou 1, leur somme étant cependant au plus égale à 1.

**2.** Mélange cristallin liquide ferroélectrique selon la revendication 1, caractérisé en ce qu'il contient un composé de formule (I) dans laquelle le groupe

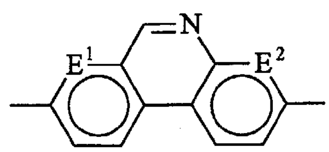

a l'une des significations suivantes:

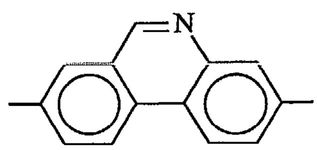

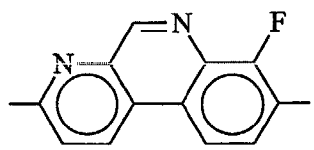

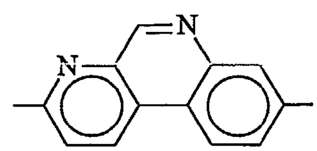

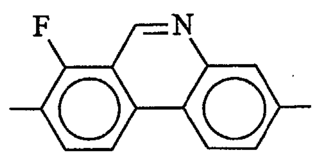

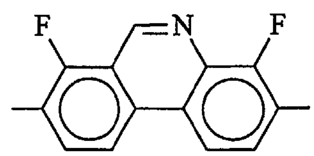

**3.** Mélange cristallin liquide ferroélectrique selon la revendication 1 ou 2, caractérisé en ce que, dans le composé de formule (I), $R^1$ et $R^2$ sont des restes alkyle linéaires ou ramifiés (avec ou sans atome de carbone asymétrique) de 1 à 16 atomes de carbone, dans lesquels un ou plusieurs groupes $CH_2$ peuvent être remplacés par -O-, cyclopropane-1,2-diyle ou $-Si(CH_3)_2-$, à condition que des atomes d'oxygène ne soient pas directement liés, et/ou un ou plusieurs atomes d'hydrogène des restes alkyle peuvent être remplacés par F.

**4.** Mélange cristallin liquide ferroélectrique selon l'une ou plusieurs des revendications 1 à 3, caractérisé en ce qu'il contient un composé de formule (I) appartenant au groupe ci-dessous, où $R^1$ et $R^2$ sont des restes alkyloxy linéaires ou ramifiés de 1 à 10 atomes de carbone dont un groupe $CH_2$, séparé du noyau par au moins deux autres groupes $CH_2$, peut être remplacé par $-Si(CH_3)_2-$:

a) 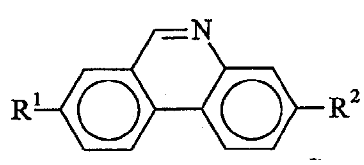

b) 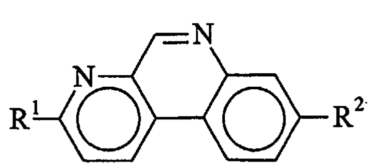

c) 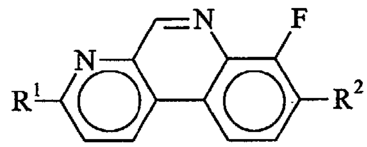

d) 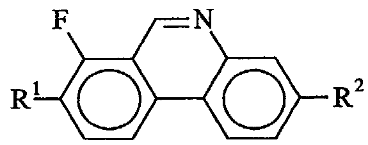

e) 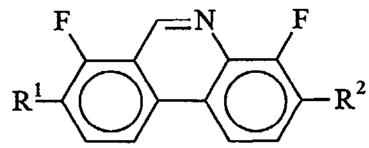

5. Mélange cristallin liquide ferroélectrique selon l'une ou plusieurs des revendications 1 à 4, caractérisé en ce qu'il est composé de 2 à 20 constituants et contient 1 à 10 composés de formule (I).

6. Mélange cristallin liquide ferroélectrique selon l'une ou plusieurs des revendications 1 à 5, caractérisé en ce qu'il contient 0,01 à 80 % en masse d'un ou plusieurs composés de formule (I).

7. Utilisation d'un mélange cristallin liquide ferroélectrique selon l'une ou plusieurs des revendications 1 à 6 dans des éléments de commutation et/ou d'affichage électro-optiques.

8. Elément de commutation et/ou d'affichage électro-optique, caractérisé en ce qu'il contient un mélange cristallin liquide ferroélectrique selon l'une ou plusieurs des revendications 1 à 6.

9. Dérivés de phénanthridine de formule (Ia)

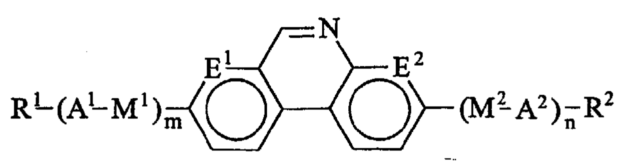

(Ia)

dans laquelle les symboles et les indices ont la signification suivante:

| | |
|---|---|
| $E^1$ et $E^2$ | sont identiques ou différents et représentent -N-, -CF- ou -CH-, |
| $R^1$ et $R^2$ | sont identiques ou différents et représentent un reste alkyle linéaire ou ramifié de 1 à 20 atomes de carbone (avec ou sans atome de carbone asymétrique), dans lequel un ou plusieurs groupes $CH_2$ peuvent être remplacés par -O-, -S-, -C≡C-, cyclopropane-1,2-diyle ou $-Si(CH_3)_2-$, à condition que des atomes d'oxygène et de soufre ne soient pas directement liés entre eux, et dans lequel un ou plusieurs atomes d'hydrogène du reste alkyle peuvent être remplacés par du fluor, ou l'un des groupes chiraux suivants (optiquement actifs ou racémiques): |

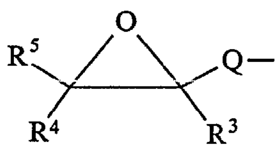
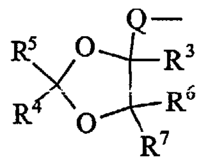
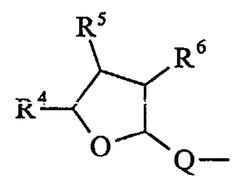

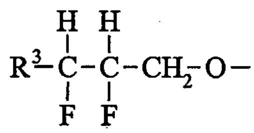
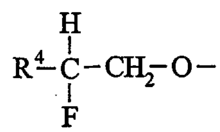 ,

$R^3$, $R^4$, $R^5$, $R^6$ et $R^7$ sont identiques ou différents et représentent un atome d'hydrogène ou un reste alkyle linéaire ou ramifié de 1 à 16 atomes de carbone (avec ou sans atome de carbone asymétrique) dans lequel un groupe $CH_2$ peut être remplacé par -O- et un ou plusieurs atomes d'hydrogène du groupe alkyle peuvent être remplacés par du fluor, $R^4$ et $R^5$ pouvant aussi former ensemble un groupe $-(CH_2)_4-$ ou $-(CH_2)_5-$ lorsqu'ils sont liés à un système dioxolane;

Q est $-CH_2-O-$, -CO-O- ou une liaison simple;

$M^1$ et $M^2$ sont identiques ou différents et représentent -CO-O-, -O-CO-, $-CH_2-O-$, $-O-CH_2-$, $-CH_2-CH_2-$, -C≡C- ou une liaison simple;

$A^1$ et $A^2$ sont identiques ou différents et représentent un groupe 1,4-phénylène dont un ou plusieurs atomes d'hydrogène peuvent être remplacés par F, trans-1,4-cyclohexylène, pyrimidine-2,5-diyle, pyridine-2,5-diyle, dont un atome d'hydrogène peut être remplacé par F, (1,3,4)-thiadiazole-2,5-diyle, 1,3-dioxane-2,5-diyle, 1,3-dithiane-2,5-diyle, 1,3-thiazole-2,4-diyle, 1,3-thiazole-2,5-diyle, thiophène-2,4-diyle ou thiophène-2,5-diyle;

m et n sont égaux à 0 ou 1, leur somme étant cependant au plus égale à 1.